# EUROPÄISCHE PATENTSCHRIFT

(11) **EP 2 736 354 B1**
(45) Veröffentlichungstag und Bekanntmachung des Hinweises auf die Patenterteilung: **16.08.2017**
(21) Anmeldenummer: 12737759.6
(22) Anmeldetag: 11.07.2012
(51) Int. Cl.: A61K 31/197, A61K 31/198, A61K 31/202, A61K 31/375, A61K 31/07, A61K 31/122, A61K 31/205, A61K 31/355, A61K 31/4415, A61K 31/519, A61K 31/714, A61K 33/30, A61K 38/06, A23L 33/115, A23L 33/15, A23L 33/16, A23L 33/175, A61K 45/06

(54) **VERWENDUNG VON CITRULLIN UND EIN KOMBINATIONSPRÄPARAT ZUR VERBESSERUNG DER MÄNNLICHEN FERTILITÄT**
USE OF CITRULLINE AND COMBINATION COMPOUND FOR IMPROVING MALE FERTILITY
UTILISATION DE CITRULLINE ET ASSOCIATION MÉDICAMENTEUSE POUR AMÉLIORER LA FÉCONDITÉ MASCULINE

(30) Priorität: 26.07.2011 AT 10932011
(43) Veröffentlichungstag der Anmeldung: 04.06.2014
(73) Patentinhaber: Gonadosan GmbH, 9244 Niederuzwil (CH)
(72) Erfinder: WOGATZKY, Birgit, 88131 Lindau (DE); WOGATZKY, Johannes, 88131 Lindau (DE)
(74) Vertreter: Burger, Hannes
(86) Internationale Anmeldenummer: PCT/EP2012/063570
(87) Internationale Veröffentlichungsnummer: WO 2013/013979

(56) Entgegenhaltungen:
- WO-A1-2004/056370
- WO-A2-2006/002096
- US-A- 5 874 471
- US-A- 6 028 107
- : "Fertilovit M", , 1 March 2010 (2010-03-01), XP055222619, Retrieved from the Internet: URL:http://www.sexovit.de/Portals/0/fertil ovit_m_de.pdf [retrieved on 2015-10-21]

## Beschreibung

Die Erfindung betrifft ein Kombinationspräparat zur Verbesserung der Spermienquantität und -qualität umfassend zumindest ein Antioxidans bzw. zumindest einen der Wirkstoffe C-Vitamine, E-Vitamine, Zink, Selen, Acetylcystein, L-Carnitin, Glutathion, Lycopen, Ubiquinon-10-Komponente deren Verwendung sowie Citrullin zur Verbesserung der Spermienqualität.

Immer mehr Paaren ist der Weg zum Wunschkind erschwert. In Industrieländern wie Deutschland und USA ist heute bereits eines von zehn Paaren ungewollt kinderlos und die Tendenz ist steigend. Eingeschränkte Fruchtbarkeit kann viele Ursachen haben. Das zunehmende Alter der Paare mit Kinderwunsch kann dabei ebenso eine Rolle spielen wie Umweltbelastung, verminderte Spermienqualität, beeinträchtigte Eizellen, ungesunde Lebensweise und anderes. Auch sogenannte "Lifestylefaktoren" stellen einen entscheidenden Faktor dar. Voraussetzung für die Fertilität ist bei Mann und Frau unter anderem eine ausreichende Versorgung des Körpers mit bestimmten Mikronährstoffen, Energiesubstraten und Vitaminen. Eine ausreichende Versorgung mit Vitaminen und gewissen Nährstoffen ist in unserer Zeit leider zunehmend schwierig. Bei erhöhten Belastungen im Alltag mit Stress, geringer Nahrungsmittelqualität und ungesunder Ernährung wird der menschliche Organismus oft unzureichend versorgt. Dadurch steht der Körper unter Dauerbelastung und das trifft besonders dort zu, wo die Zellen am empfindlichsten sind - im Bereich der Ei- und Samenzellen. Die männlichen Spermien werden träge, Eizellen "verpassen" ihren Eisprung und somit kann die Fruchtbarkeit unter Fehlernährung leiden. Es wurde bereits in mehreren Studien nachgewiesen, wie sehr die richtige Ernährung zur Erfüllung des Kinderwunsches beiträgt.

Während die Frau bereits mit den Eizellen geboren wird, werden die Spermien beim Mann ein Leben lang nachgebildet. Bei jedem zweiten ungewollt kinderlosem Paar ist der Mann ursächlich mitbeteiligt und in jedem dritten Fall liegt die Ursache sogar alleine beim Mann. In den letzten Jahrzehnten nahm die Qualität der männlichen Zeugungsfähigkeit kontinuierlich ab. Einer der häufigsten Gründe männlicher Zeugungsunfähigkeit ist dabei eine eingeschränkte Spermienqualität im Sinne einer Oligozoospermie (zu wenige Spermien), Asthenozoospermie (zu schlecht bewegliche Spermien) oder Teratozoospermie (zu viele formveränderte Spermien). Nicht selten findet man alle drei Einschränkungen sogar bei ein und demselben Patienten (OAT-Syndrom). Neben einer abnehmenden Spermienanzahl spielt aber auch die Integrität der in den Spermien transportierten DNA eine wichtige Rolle für die Erfüllung des Kinderwunsches. Ein Hauptverursacher von DNA Schäden im Spermium ist oxidativer Stress. In den letzten zehn Jahren ist oxidativer Stress daher als Auslöser für Infertilität immer mehr in den Focus des Interesses gerückt (Agarwal, 2004). Unter oxidativem Stress versteht man die Schädigung von Zellen, Geweben oder gar Organen durch erhöhte Mengen an sogenannten reaktiven Sauerstoffspezies (ROS) (Moller et al, 1996; Sharma and Agarwal, 1996; Saleh et al, 2003). Übersteigt die Menge an reaktiven Sauerstoffspezies die Kapazitäten der körpereigenen antioxidativen Abwehrmechanismen, so entsteht der oben genannte oxidative Stress (OS). 30 - 80 % aller infertilen Männer haben eine erhöhte Menge an ROS im Seminalplasma (Agarwal, 2004). 2009 konnten Varghese et al. zudem eine starke Korrelation zwischen Asthenospermie und reduzierten Antioxidantien in der Samenflüssigkeit nachweisen. Insbesondere bei Männern, die überschießende Mengen von reaktiven Sauerstoffspezies produzieren, kommt es zu einer Schädigung der normalen Spermien (Aitken et al., 1998, Alvarez et al., 2002, Saleh et al., 2002, Agarwal et al., 2003). Spermien sind besonders anfällig gegenüber Angriffen durch ROS, da sie keine eigenen zytoplasmatischen Abwehr- und Reparaturmechanismen besitzen (Donnelly et al, 1999; Saleh and Agarwal, 2002). Hinzu kommt, dass sie eine Plasmamembran mit besonders vielen mehrfach ungesättigten Fettsäuren haben. Diese sind gute Angriffspunkte für ROS. (Agarwal et al, 1994; Kobayashi et al, 2001; Zalata et al, 2004). Antioxidantien, die mit der Nahrung aufgenommen werden, stellen einen wichtigen Teil des menschlichen antioxidativen Schutzsystems - insbesondere auch im Bereich der Spermiogenese - dar. Obst und Gemüse sind eine Hauptquelle für die verschiedenen Antioxidantien, können aber unter den heutigen zunehmenden Belastungen und dem dadurch resultierenden erhöhten oxidativen Stress, selbst bei ausgeglichener Ernährung kaum noch im erforderlichen Maß zugeführt werden. Erschwerend kommt hinzu, dass insbesondere Männer sich häufig nicht besonders bewusst und gesund ernähren. Laut einer Umfrage unter männlichen Patienten in reproduktionsmedizinischer Behandlung glaubt die Mehrzahl der Männer von sich selbst, sie sei sehr gut ernährt, auch wenn sie nach eigener Auskunft nicht mehr als ein bis maximal zwei Portionen Obst und Gemüse pro Tag zu sich nahmen. Die von den Gesundheitsbehörden vieler Staaten empfohlenen 5 Portionen Obst und Gemüse aber erreicht kaum ein Mann (Wogatzky et al, Vortag beim 4. Internationalen Kongress der Jordanischen Gesellschaft für Fruchtbarkeit und Genetik, 20. - 22. April 2011). In einer spanischen Studie aus dem Jahre 2009 konnte gezeigt werden, dass Männer, die mit der Nahrung wenige Antioxidantien zu sich nahmen, eine durchschnittlich schlechtere Samenqualität aufwiesen (Mendiola et al, 2009).

So ist beispielsweise das Präparat Fertilovit^{®} M der Anmelderin bekannt. Fertilovit^{®} M enthält eine Kombination von Vitaminen und anderen Vitalstoffen, die die Spermien bei ihrer Reifung unterstützen und zusätzlich durch Antioxidantien in Depotform rund um die Uhr vor oxidativem Stress schützen. Fertilovit^{®} M ist ein diätetisches Lebensmittel zur Verbesserung der Spermienqualität umfassend C-Vitamin, E-Vitamine, Folsäure, Zink, Selen, Acetylcystein, L- Carnitin, Glutathion, Ubiquinon-10-Komponente und Lycopen. Fertilovit^{®} M für den Mann verbessert durch seine spezifische Zusammenstellung körpereigener Substanzen die Samenqualität.

Auch die US 6,028,107 A beschreibt ein Verfahren zur Verbesserung der Gesundheit, wobei die Plasmakonzentration von Arginin durch die Gabe von Citrullin als Vorläufer vervielfacht wird. Unter anderem können auch Männer mit Oligospermie bzw. verringerter Spermienmotilität behandelt werden. Als zweiter aktiver Wirkstoff kann L-Ascorbinsäure enthalten sein.

Die US 5,874,471 A offenbart die Verwendung von Citrullin zur Behandlung von Oligozoospermie, indem Citrullin als Vorläufersubstanz für die Bildung von L-Arginin verabreicht wird, um einen höheren Plasmaspiegel von L-Arginin zu erreichen.

Ferner offenbart die WO 2004/056370 A1 die Verwendung von Omega-3- Fettsäuren, wie Eicosapentaensäure und/oder Docosahexaensäuren, zusammen mit einem Antioxidans, wie z.B. Ascorbinsäure, zur Behandlung männlicher Infertilität.

Die WO 2006/002096 A2 beschreibt die Verwendung von L-Citrullin zur Behandlung von Krankheiten, u.a. Infertilität. Niedrige Dosen von L-Citrullin sind besonders wirksam in der Kombination mit L-Carnitin in der Behandlung von männlicher Infertilität. Zur Behandlung weiblicher Infertilität wird L-Citrullin vorzugsweise in Kombination mit Mönchspfeffer und/oder grünem Tee, der auch antioxidative Wirkung hat, verwendet.

Aufgabe der vorliegenden Erfindung ist es daher, einen Wirkstoff bzw. ein Kombinationspräparat zur Verfügung zu stellen, welches die Spermienqualität des Mannes weiter verbessert und die Spermienquantität erhöht.

Die Aufgabe der Erfindung wird durch ein Kombinationspräparat umfassend zumindest die Wirkstoffe C-Vitamin, E-Vitamine, Zink, Selen, Acetylcystein, L-Carnitin, Glutathion, Ubiquinon-10-Komponente, Lycopen und Citrullin, wobei Citrullin in einer Tagesdosis von 200 mg bis 400 mg verabreicht wird und/oder Omega-3-Fettsäuren, zur Anwendung in einem Verfahren für die Behandlung von Oligozoospermie, Asthenozoospermie und/oder Teratozoospermie gelöst. Citrullin zeigt eine sehr gute Bioverfügbarkeit, wodurch der Organismus die Polyamine Spermin und Spermidin, die in hoher Konzentration in der Spermienflüssigkeit vorkommen, sowie die Protamine, die für die Verpackung der Spermien - DNA notwendig sind und die DNA stabilisieren, in ausreichender Menge produzieren kann. Omega-3-Fettsäuren zeigen einen positiven Effekt auf die Fusion von Ei- und Samenzelle, sowie den Prostaglandin- und Steroidhormonstoffwechsel. Bei vielen Patienten (ungefähr 70 %) verbessert die routinemäßige Gabe zusätzlicher Antioxidantien des Kombinationspräparats die Spermienqualität deutlich. Die Patienten wiederum, die von einer solchen Maßnahme nicht profitieren, haben auf der anderen Seite keine negativen Nebenwirkungen zu befürchten. C-Vitamin des Kombinationspräparats unterbricht die radikalische Kettenreaktion und neutralisiert so Hydroxyl-, Hyperoxid-, und Wasserstoffdioxid-Radikale. C-Vitamin wirkt als Hauptantioxidans im Seminalplasma fertiler Männer; hier ist die Konzentration sogar zehnmal höher als im Blutplasma. In der Samenflüssigkeit unfruchtbarer Männer findet sich meist nur eine geringe Menge.

E-Vitamine werden in der Zellmembran lokalisiert und unterbrechen hier sehr effektiv die radikalische Kettenreaktion und neutralisieren so vor allem H₂O₂, OH und O₂-. Dadurch schützen sie die Zellmembran vor Lipidoxidation. Besonders vorteilhaft dabei erweist sich, dass die Spermienmotilität bei 60 % der Patienten mit Asthenozoospermie verbessert wird.

Durch Selen des Kombinationspräparats kann bei OAT-Patienten die Spermienbeweglichkeit, Lebensfähigkeit und Morphologie der Spermien verbessert werden. Zudem wird die Spermien-DNA vor oxidativem Stress durch Förderung der Protamin-Verpackung der DNA geschützt und Selen hat auch antioxidative Wirkung als Bestandteil der Glutathion Peroxidase. Selen arbeitet auch synergistisch mit Vitamin E.

Zink ist ein wesentlicher Bestandteil der Spermatozyten. Steht dem Körper nicht genügend Zink für die Spermienproduktion zu Verfügung, so verringert sich diese deutlich. Auch die Motilität der Samenzellen nimmt ab, weil Zink zur Bindung des Samenkopfes an den Samenschwanz beiträgt. Durch das zusätzliche Zink des Kombinationspräparats kommt es bei infertilen Männern zu einem verbesserten Spermiogramm. Die Kombination von Zink mit Folsäure führt zu einer 74 % Erhöhung der Spermienanzahl. Zudem führt eine Behandlung über einen Zeitraum von 3 Monaten zu einer deutlichen Verbesserung bei Astenozoospermie. Zink, das an fast allen biochemischen Prozessen im Körper beteiligt ist, unterstützt die Wirkung des erfindungsgemäßen Kombinationspräparats.

L-Carnitin des Kombinationspräparats fungiert als Rezeptormolekül für aktivierte Fettsäuren im Zytosol und in den Mitochondrien (im Wechselspiel mit Azetyl-CoA) und ist ein wichtiges Energiesubstrat für die Spermatozoen. Carnitine sind aber auch Antioxidantien, die die Menge an reaktiven Sauerstoffspezies dadurch senken, dass sie überschüssiges intrazelluläres Azetyl-CoA entfernen. Dieses steigert sonst die ROS Produktion durch die Mitochondrien. Durch L-Carnitin wird auch die Spermienmotilität verbessert. Carnitin, genauer L-Carnitin, spielt eine essentielle Rolle im Energiestoffwechsel tierischer Zellen.

Durch die Beimengung von Glutathion zum Kombinationspräparat wird die Beweglichkeit von Spermien signifikant erhöht. Zudem verringert Glutathion DNA-Schäden durch ROS. Das Tripeptid Glutathion (Cystein, Glutamat, Glycin) ist das häufigste Antioxidans im menschlichen Körper. Es schützt Lipide, Proteine und Nukleinsäuren vor oxidativem Stress. Glutathion ist in fast allen Zellen in hoher Konzentration enthalten und gehört zu den wichtigsten als Antioxidans wirkenden Stoffen im Körper. Gleichzeitig ist es eine Reserve für Cystein. In Kombination mit Vitamin E und Selen bildet es die Glutathion-Peroxidase. Eine Gabe von Glutathion als Nahrungsergänzungsmittel allein führt jedoch nicht immer zum gewünschten Erfolg, da die orale Gabe nur zu einer mäßigen Steigerung des im Körper zirkulierenden Glutathion führt. Jedoch kann durch die orale Gabe von N-Acetylcystein des Kombinationspräparats, einem wichtigen Vorläufermolekül von Glutathion, der Glutathion-Gehalt in Zellen effektiv erhöht werden. Oral verabreichtes Acetylcystein über einen Zeitraum von 3 Monaten führt zu einer signifikanten Erhöhung der gesamt antioxidativen Kapazität im Plasma. Dies geht einher mit geringerer Viskosität und somit mit verbesserter Beweglichkeit des Samens.

Lycopen, auch Lycopin oder Leukopin, zählt zu den Antioxidantien und gilt als Radikalfänger, d.h., es kann bestimmte reaktionsfreudige Moleküle im menschlichen Körper unschädlich machen. Lycopen ist ein Carotinoid mit sehr guten antioxidativen und antientzündlichen Eigenschaften. Durch diese antientzündlichen Eigenschaften reduziert es durch die Beimengung im Kombinationspräparat die seminale Neuproduktion von ROS durch Leukozyten.

Die Beimengung der Ubiquinon-10-Komponente (Coenzym Q10) im Kombinationspräparat führt zu einer Erhöhung der Spermienmenge und zu einer Verbesserung der Beweglichkeit. Coenzym Q10 (CoQ10) wird vom Körper unter anderem für die Energiegewinnung in den Mitochondrien benötigt. In Spermien findet man es daher vor allem im Mittelstück. Es hat aber auch antioxidative Eigenschaften, indem es Vitamin E recycelt und dessen pro-oxidative Aktivität unterbindet. Die Gewebekonzentration von Coenzym Q10 nimmt mit zunehmendem Alter ab, womit durch das Kombinationspräparat diese Abnahme ausgeglichen werden kann. Durch die Gabe des Kombinationspräparats mit oralem CoQ-10 an infertile männliche Probanden wird die Fertilisationsrate deutlich erhöht. CoQ-10 verhindert auch die Bildung von H₂O₂ in der Samenflüssigkeit.

Folsäure unterstützt die Protamin-Verpackung der Spermien-DNA. Durch eine folsäurearme Ernährung des Mannes steigt die Zahl der Spermien mit chromosomalen Abberationen. Männer, die reichlich Folsäure über ihre Ernährung oder über Vitaminpräparate zu sich nehmen, haben signifikant seltener Spermien mit einer Disomie des X-Chromosoms (Klinefelter) oder des Chromosoms 21 (Down-Syndrom) oder eine Nullosomie des X-Chromosoms (Turner). Nach Berücksichtigung anderer Faktoren wie Alter, Alkoholkonsum und Komorbidität haben Männer mit der höchsten Folsäurezufuhr (722-1150 µg/d) zu 20-30 Prozent weniger Spermien mit Aneupliodie im Ejakulat als Männer mit der geringsten Folsäurezufuhr. Pro 100 µg Folsäure sinkt das Risiko um 3,6 Prozent. Weiterhin fördert Folsäure auch den Homocysteinstoffwechsel, was einer Hyperhomocysteinämie entgegenwirkt und sich so zusätzlich vorteilhaft auf die Spermiogenese auswirkt.

Das Kombinationspräparat verbessert durch hochwertige Omega-3-Fettsäuren die Membranstruktur der Spermien, was insbesondere für die Verschmelzung von Samenzelle und Eizelle während der eigentlichen Befruchtung von Bedeutung ist, weil für die gesunde Spermienfunktion eine ausgeglichene Membranstruktur der Zellen eine wichtige Rolle spielt. Zeugungsunfähige Männer haben im Vergleich zu fruchtbaren Männern einen geringeren Anteil mehrfach ungesättigter Fettsäuren, und zwar insbesondere einen geringeren Anteil von Omega-3-Fettsäuren. Dies geht einher mit reduzierter Spermienanzahl, Beweglichkeit und Struktur der Spermien. Eine Zufuhr von Omega-3-Fettsäuren kann diesen Problemen entgegen wirken. Als positiver Nebeneffekt gilt die Schutzwirkung von Omega-3-Fettsäuren für die Prostata und das Herz-Kreislaufsystem. Die Membran wird zum großen Teil von Fetten gebildet. Durch veränderte Ernährungsbedingungen haben sich in den vergangenen Jahren die relativen Anteile verschiedener Fettsäuren in den Zellmembranen nachweislich verändert. Insbesondere die essentiellen mehrfach ungesättigten Omega-3-Fettsäuren sind relativ gesehen zu wenig vorhanden. Dies hat Einfluss auf Vorgänge wie zum Beispiel die Fusion von Ei- und Samenzelle, sowie den Prostaglandin- und Steroidhormon-Stoffwechsel. Während gezeigt werden konnte, dass sich gesättigte und auch einfach ungesättigte Fette schädlich auf die Spermienqualität auswirken, sind sowohl Omega-6 als auch Omega-3-Fettsäuren sehr gut für die Spermienqualität. Von Bedeutung ist auch das Verhältnis von Omega-3-Fettsäuren zu Omega-6-Fettsäuren.

Im Kombinationspräparat kann auch Citrullin enthalten sein. Citrullin wird vom Körper für die Synthese von Arginin genutzt. Eine wichtige Eigenschaft des Arginin ist, dass es wiederum Vorstufe des Neurotransmitters Stickstoffmonoxid (NO) ist. Das Enzym Stickstoffmonoxid-Synthase katalysiert die Umsetzung der Aminosäure Arginin in den Endothelium derived relaxing Factor (EDRF) - eine andere Bezeichnung für Stickstoffmonoxid. Dieser diffundiert in die Muskelschichten der Gefäße bzw. des Corpus cavernosum. Dort führt er über die Aktivierung des Enzyms Guanylatcyclase zu einer erhöhten Ausschüttung von cyclischem Guanosinmonophosphat (cGMP). Die Erhöhung von cGMP bewirkt eine Erschlaffung der glatten Muskulatur und damit ein Herabsetzen des Gefäßtonus. Somit löst das gasförmige NO im Herzmuskel und in glatten Gefäßmuskeln eine Entspannung aus.

Die Aminosäure L-Arginin verbessert so über die Freisetzung von Stickstoffmonoxid die Durchblutung und Sauerstoffversorgung der koronaren und peripheren Blutgefäße, hemmt die Klumpenbildung bei Thrombozyten und senkt den Blutdruck. Durch diesen Mechanismus kann zudem die Erektionsfähigkeit des Gliedes gesteigert werden.

Arginin ist darüber hinaus für die normale Spermatogenese notwendig. Aus Arginin bildet der Körper nämlich die Polyamine Spermin und Spermidin, die in hoher Konzentration in der Spermienflüssigkeit vorkommen, sowie die Protamine, die für die Verpackung der Spermien-DNA notwendig sind und die DNA stabilisieren.

Das Kombinationspräparat führt bei infertilen Männern bereits nach 6 bis 8 Wochen zu einer Steigerung der Spermienmenge und -beweglichkeit. Arginin hat allerdings den Nachteil, oral verabreicht keine optimale Bioverfügbarkeit zu haben. Die Aminosäure L-Citrullin ist eine wichtige Vorstufe für die Herstellung von Arginin im Körper wird aber im Gegensatz zu Arginin sehr viel besser enteral resorbiert. Sie hat somit eine sehr gute Bioverfügbarkeit. Oral eingenommen, wird L-Citrullin über den Darm in den Blutkreislauf aufgenommen und vom Körper in L-Arginin umgewandelt. Studien haben gezeigt, dass eine Supplementierung mit Citrullin den Arginin Spiegel effektiver anheben kann, als wenn die gleiche Dosis Arginin direkt eingenommen würde. Da der Körper Citrullin zeitverzögert in Arginin umwandelt, agiert Citrullin gemäß der Retard-Funktion als Verweildauer-Verlängerer und erhöht den Arginin-Spiegel im Blut über längere Zeit. So profitiert der Organismus von einer spürbar längeren Arginin-Wirkung. Die Verwendung von Citrullin zur Herstellung eines Kombinationspräparats umfassend zumindest Antioxidantien zur Verbesserung der Spermienqualität erweist sich auch als vorteilhaft.

Günstig zeigt sich auch ein Verhältnis von Eicosapentaensäure zu Docosahexaensäure von 3 zu 2, weil dadurch die Spermienanzahl erhöht werden kann und die Beweglichkeit und Struktur der Spermien verbessert werden.

Durch die Auswahl der Mengen der Wirkstoffe C-Vitamine in Mengen von 1 bis 20 Gew.-%, insbesondere 5 bis 15 Gew.-%, vorzugsweise 6 bis 10 Gew.-%, E-Vitamine in Mengen von 1 bis 20 Gew.-%, insbesondere 5 bis 15 Gew.-%, vorzugsweise 6 bis 10 Gew.-%, B9-Vitamine in Mengen von 0,005 bis 2 Gew.-%, insbesondere 0,009 bis 1 Gew.-%, vorzugsweise 0,01 bis 0,1 Gew.-%, Zink in Mengen von 0,5 bis 5 Gew.-%, insbesondere 0,8 bis 3,5 Gew.-%, vorzugsweise 1,2 bis 2,5 Gew.-%, Selen in Mengen von 0,001 bis 1 Gew.-%, insbesondere 0,004 bis 0,5 Gew.-%, vorzugsweise 0,006 bis 0,1 Gew.-%, Acetylcystein in Mengen von 0,5 bis 10 Gew.-%, insbesondere 1 bis 6 Gew.-%, vorzugsweise 2,5 bis 4,5 Gew.-%, Carnitin in Mengen von 3 bis 30 Gew.-%, insbesondere 5 bis 25 Gew.-%, vorzugsweise 10 bis 23 Gew.-%, Glutathion in Mengen von 0,5 bis 15 Gew.-%, insbesondere 1 bis 10 Gew.-%, vorzugsweise 3 bis 6 Gew.-%, Lycopen in Mengen von 0,05 bis 3 Gew.-%, insbesondere 0,08 bis 1 Gew.-%, vorzugsweise 0,2 bis 0,1 Gew.-%, und eine Ubiquinon-10-Komponente in Mengen von 0,05 bis 10 Gew.-%, insbesondere 0,1 bis 5 Gew.-%, vorzugsweise 0,8 bis 3 Gew.-%, und Omega-3-Fettsäuren in Mengen von 5 bis 50 Gew.-%, insbesondere 10 bis 40 Gew.-%, vorzugsweise 20 bis 35 Gew.-%, und/oder Citrullin in Mengen von 5 bis 40 Gew.-%, insbesondere 8 bis 30 Gew.-%, vorzugsweise 12 bis 20 Gew.-%, bezogen auf das Gesamtgewicht der therapeutisch wirksamen Wirkstoffe des Kombinationspräparats werden die vorab beschriebenen positiven Wirkungen der Einzelwirkstoffe in ein optimales Verhältnis zueinander gestellt und somit die Spermienqualität und Anzahl am effektivsten gesteigert.
Durch die Beimengung von B6-Vitaminen und B 12-Vitaminen im Kombinationspräparat wird die Gefäßfunktion durch Senkung der Homocysteinsäurewerte unterstützt und somit eine ausreichende Produktion der Spermien und die Spermatogenese unterstützt.

Kupfer ist Bestandteil vieler Enzyme (Metalloenzyme) und daher ein lebensnotwendiges Spurenelement und kann daher auch eine optimale Spermienproduktion unterstützen.

Durch das große antioxidative Potential der weiteren Carotinoide wird die Spermienqualität ebenfalls verbessert.

Vorteilhaft erweist sich auch die Zugabe von Kupfer in Mengen von 0,001 bis 1 Gew.-%, insbesondere 0,003 bis 0,5 Gew.-%, vorzugsweise 0,05 bis 0,1 Gew.-%, B6-Vitamine in Mengen von 0,05 bis 3 Gew.-%, insbesondere 0,08 bis 1 Gew.-%, vorzugsweise 0,2 bis 0,1 Gew.-%, B12-Vitamine in Mengen von 0,001 bis 1 Gew.-%, insbesondere 0,003 bis 0,5 Gew.-%, vorzugsweise 0,05 bis 0,1 Gew.-%, weitere Carotinoide, wie β-Carotin, α-Carotin, β-Cryptoxanthin, Lutein und Zeaxanthin in Mengen von 0,05 bis 3 Gew.-%, insbesondere 0,08 bis 1 Gew.-%, vorzugsweise 0,2 bis 0,1 Gew.-%, bezogen auf das Gesamtgewicht der therapeutisch wirksamen Wirkstoffe des Kombinationspräparats, weil dadurch die vorab beschriebenen vorteilhaften Wirkungen sich kumulativ auf die Entwicklung der Spermien auswirken. Durch die beschriebene Zusammensetzung des Kombinationspräparats werden die Zusammenwirkung der Einzelsubstanzen untereinander optimiert und die Synergieeffekte zusätzlich verstärkt. Zudem kann eine ausgeglichene Wechselwirkung mit den anderen Substanzen erreicht werden.

Das Kombinationspräparat kann sowohl als Arzneimittel bzw. bilanzierte Diät als auch als Nahrungsergänzungsmittel verwendet werden, wodurch für den Anwender bei der Zulassung als Arzneimittel bzw. bilanzierte Diät gewährleistet ist, dass das Präparat zumindest eine klinische Studie durchlaufen hat und bei der Zulassung als Nahrungsergänzungsmittel ein einfacher und gegebenenfalls diskreter Zugang zum Präparat gegeben ist.

Vorteilhaft erweist sich zudem Citrullin zur Behandlung von Oligozoospermie, Asthenozoospermie und/oder Teratozoospermie zu verwenden. Vorteilhaft erweist sich Citrullin in Tagesdosen von 100 mg bis 1000 mg, insbesondere 150 mg bis 500 mg, vorzugsweise 200 mg bis 300 mg, zu verabreichen, womit gewährleistet ist, dass ein angemessener Spiegel von Citrullin im Organismus vorhanden ist und somit eine normale Spermatogenese erfolgen kann. Dadurch wird eine hohe Spermienmenge und gute Spermienbeweglichkeit gefördert.

Citrullin ist in Mengen von 2 bis 50 Gew.-%, insbesondere 5 bis 35 Gew.-%, vorzugsweise 10 bis 20 Gew.-%, bezogen auf das Gesamtgewicht der therapeutisch wirksamen Wirkstoffe des Kombinationspräparats enthalten, wodurch einerseits eine ausreichende Wirkung von Citrullin gegeben ist aber andererseits auch keine Gefahr zur Überdosierung besteht. Durch den gewählten Gewichtsanteil ist auch eine gute Verarbeitbarkeit in der Herstellung des Kombinationspräparats gegeben.

Die Erfindung beschreibt Citrullin zur Anwendung in einem therapeutischen Verfahren zur Behandlung eines eingeschränkten Spermiogramms. Die Kombination mit Antioxidantien zeigt sich besonders vorteilhaft. Ein Antioxidans im Sinne der Erfindung ist eine chemische Verbindung, die eine unerwünschte Oxidation anderer Substanzen gezielt verhindert. Antioxidantien haben große physiologische Bedeutung durch ihre Wirkung als Radikalfänger. Sie inaktivieren im Organismus reaktive Sauerstoffspezies (ROS), deren Vorkommen im Übermaß zu oxidativem Stress führt. Oxidativer Stress gilt als mitverantwortlich für den Alterungsprozess und die Beeinträchtigung der Fruchtbarkeit und wird auch in Zusammenhang mit der Entstehung einer Reihe von Krankheiten gebracht.

Ferner beschreibt die Erfindung Citrullin zur Anwendung in einem Verfahren zur Herstellung eines Kombinationspräparats zur Behandlung eines eingeschränkten Spermiogramms. Die Wirkungen der verschiedenen Wirkstoffe des Kombinationspräparats wurden bereits vorab beschrieben. Das Kombinationspräparat erweist sich durch die gezielte Auswahl der Wirkstoffe in den entsprechenden Mengen als besonders vorteilhaft zur Behandlung eines eingeschränkten Spermiogramms bei Oligozoospermie, Asthenozoospermie und/oder Teratozoospermie. Besonders vorteilhaft zeigt sich eine Kombination von C-Vitamine, E-Vitamine, Zink, Selen, Acetylcystein, L-Carnitin, Glutathion, Lycopen, Ubiquinon-10-Komponente mit Citrullin und/oder Omega-3-Fettsäuren.

Als Omega-3-Fettsäuren werden vorzugsweise Eicosapentaensäure (EPA) und Docosahexaensäure (DHA) verwendet. Es kann aber auch α-Linolensäure (ALA) als Omega-3-Fettsäure im Kombinationspräparat verwendet werden. Vorzugsweise wird für die Herstellung des Kombinationspräparats Fischöl verwendet, wobei 18 % DHA und 12 % EPA sind. Es können Omega-3-Fettsäuren sowohl tierischer als auch pflanzlicher Herkunft verwendet werden.

Das erfindungsgemäße Kombinationspräparat ist speziell für die Bedürfnisse des männlichen Organismus bei Kinderwunsch. Es kann sowohl ein Nahrungsergänzungsmittel, ein diätetisches Lebensmittel für besondere medizinische Zwecke (ergänzende bilanzierte Diät), als auch ein Arzneimittel sein.

Ferner zeigt das erfindungsgemäße Kombinationspräparat eine speziell abgestimmte Mischung von Antioxidantien, teilweise in Depotform, und anderer für die Fortpflanzungszellen wichtiger Nähr- und Mineralstoffe. So wird beispielsweise Vitamin C als Retardform beigemengt. Aus dem Stand der Technik sind Zubereitungen für eine retardierte Freisetzung von Wirkstoffen beschrieben. Beispielsweise kann Vitamin C mit Hydroxypropylmethylcellulose beschichtet sein. Somit steht eine optimale Versorgung des Mannes zur Verfügung. Selbstverständlich können auch andere aus dem Stand der Technik bekannte Methoden für die zeitversetzte Freisetzung von Vitamin C angewendet werden.

Die Wirkmechanismen der einzelnen Substanzen im erfindungsgemäßen Kombinationspräparat sind komplex. Neben Nährstoffgabe und Enzyminduktion ist ein wesentlicher Wirkmechanismus die gezielte Reduktion der Sauerstoffradikale.

Die Kombination der aufeinander abgestimmten Wirkstoffe in dem erfindungsgemäßen Kombinationspräparat hat in Untersuchungen gezeigt, dass es bei Männern zu einer deutlichen Verbesserung ihrer Fruchtbarkeit kommt.

Die vorab beschriebenen Mechanismen funktionieren vorzugsweise in Kombination der Wirkstoffe und durch Unterstützung mehrerer Antioxidativa.

Durch den synergistischen Effekt der einzelnen Wirkstoffe in den ausgewählten Mengenbereichen kann die Wirksamkeit bezüglich Spermienqualität und -quantität des erfindungsgemäßen Kombinationspräparats erzielt werden.

Die Verwendung von Citrullin, gegebenenfalls in Kombination mit zumindest einem Antioxidans bzw. Omega-3-Fettsäuren mit zumindest einem Antioxidans als auch des erfindungsgemäßen Kombinationspräparats wird vorzugsweise für die orale Verabreichung empfohlen. Das Präparat kann sowohl als Human- als auch Veterinärprodukt verwendet werden.

In der nachfolgenden Tabelle sind die Mengenbereiche der empfohlenen Tagesdosis der einzelnen für die Erfindung relevanten Wirkstoffe angegeben. Vorzugsweise wird das erfindungsgemäße Kombinationspräparat als zwei Kapseln pro Tag eingenommen. Es können allerdings die Wirkstoffmengen so gewählt werden, dass nur eine Kapsel pro Tag erforderlich ist um die empfohlene Dosierung zu erreichen.

| **Wirkstoff** | **empfohlener Mengenbereich in mg pro Tag** |
|---|---|
| Vitamin C | 50 -200 |
| Vitamin E | 10 - 120 |
| Folsäure | 0,1 - 0,7 |
| Zink | 5 - 50 |
| Selen | 0,02 - 0,2 |
| Acetylcystein | 10 - 100 |
| Carnitin | 10 - 500 |
| Glutathion | 10 - 100 |
| Lycopen | 1 - 15 |
| Ubiquinon-10 Komponente | 1 - 50 |
| Omega-3-Fettsäuren | 100 - 1000 |
| Citrullin | 1 - 600 |
| Kupfer | 0,05 - 10 |
| Vitamin B 12 | 0,01 - 1 |
| Vitamin B6 | 1 - 10 |
| Carotinoide | 0,1 - 10 |

Es sind auch Exzipienten, wie Hilfsstoffe, Füllstoffe, Trägerstoffe, Bindemittel, etc., welche zur technischen Verarbeitbarkeit des erfindungsgemäßen Kombinationspräparats erforderlich sind, enthalten. Diese Exzipienten haben jedoch keine therapeutische Wirksamkeit und keine positiven Effekte auf die Spermien und sind dementsprechend auch keine therapeutisch wirksamen Wirkstoffe im Sinne der Erfindung.

Im nachfolgenden sind Ausführungsbeispiele zur Zusammensetzung der therapeutisch wirksamen Wirkstoffe in mg pro Tagesdosis des erfindungsgemäßen Kombinationspräparats angeführt, das die vorteilhaften Wirkungen erzielt.

| **Wirkstoff** | **1** | **2** | **3** | **4** | **5** | **6** | **7** | **8** | **9** | **10** |
|---|---|---|---|---|---|---|---|---|---|---|
| Vitamin C | - | 50 | 120 | 150 | 100 | 180 | 200 | 100 | 80 | 100 |
| Vitamin E | - | 10 | 20 | 50 | 100 | 100 | 120 | 100 | 60 | 80 |
| Folsäure | - | 0,1 | 0,3 | 0,3 | 0,5 | 0,6 | 0,7 | 0,5 | 0,4 | 0,5 |
| Zink | - | 5 | 10 | 15 | 25 | 40 | 50 | 25 | 30 | 10 |
| Selen | - | 0,02 | 0,05 | 0,07 | 0,1 | 0,1 | 0,2 | 0,1 | 0,09 | 0,15 |
| Acetylcystein | - | 10 | 40 | 30 | 50 | 90 | 100 | 50 | 80 | 80 |
| Carnitin | - | 10 | 100 | 200 | 300 | 400 | 500 | 300 | 250 | 350 |
| Glutathion | - | 10 | 20 | 25 | 50 | 80 | 100 | 50 | 90 | 70 |
| Lycopen | - | 1 | 2 | 3 | 4 | 2,5 | 15 | 4 | 13 | 10 |
| Ubiquinon-10 Komponente | - | 1 | 3 | 10 | 15 | 15 | 50 | 15 | 30 | 40 |
| Omega-3-Fettsäuren | - | 100 | 200 | 400 | 500 | 350 | 1000 | 500 | 700 | 900 |
| Citrullin | 500 | 100 | 300 | 800 | 300 | 600 | 600 | 200 | 60 | 10 |
| Kupfer | - | - | - | - | - | 0,5 | - | 1 | - | - |
| Vitamin B 12 | - | - | 0,05 | - | - | 0,01 | - | 0,09 | - | - |
| Vitamin B6 | - | - | - | - | - | 1 | - | 3 | 2 | - |
| Carotinoide | | - | - | 5 | - | 5 | - | 3 | - | 3 |
| Füll- und Trägerstoffe | ✔ | ✔ | ✔ | ✔ | ✔ | ✔ | ✔ | ✔ | ✔ | ✔ |

In Versuchen konnte nachgewiesen werden, dass das Ausführungsbeispiel 5 besonders gute Wirksamkeit zeigt. So kommt es mit einer Zusammensetzung gemäß Ausführungsbeispiel 5 zu einer Verbesserung des Spermiogramms und zu einer um ca. 20 % erhöhten Befruchtungsrate. Generell wurde nachgewiesen, dass mit dem erfindungsgemäßen Präparat die Spermienquantität und -qualität bei über 70 % der Männer signifikant verbessert wird.

In der unten stehenden Tabelle sind beispielhaft die Zusammensetzungen der Wirkstoffe in absoluten Zahlen zweier erfindungsgemäßer Kombinationspräparate, die die vorteilhaften Wirkungen entfalten, beschrieben.

| **Wirkstoff** | **Menge in mg pro Tages-dosis** | **Gew.-%** | **Menge in mg pro Tagesdosis** | **Gew.-%** |
|---|---|---|---|---|
| Vitamin C | 100 | 7,43715603 | 100 | 7,39814602 |
| Vitamin E | 100 | 7,43715603 | 100 | 7,39814602 |
| Folsäure (Vitamin B9) | 0,5 | 0,03718578 | 0,5 | 0,03699073 |
| Zink | 25 | 1,85928901 | 25 | 1,84953651 |
| Selen | 0,1 | 0,00743716 | 0,1 | 0,00739815 |
| Acetylcystein | 50 | 3,71857802 | 50 | 3,69907301 |
| Carnitin | 300 | 22,3114681 | 300 | 22,1944381 |
| Glutathion | 50 | 3,71857802 | 50 | 3,69907301 |
| Lycopen | 4 | 0,29748624 | 4 | 0,29592584 |
| Ubiquinon-10 Komponente | 15 | 1,1155734 | 15 | 1,1097219 |
| Omega-3-Fettsäuren | 500 | 37,1857802 | 500 | 36,9907301 |
| Citrullin | 300 | 14,8743121 | 300 | 14,796292 |
| Kupfer | - | 0 | 3 | 0,22194438 |
| Vitamin B 12 | - | 0 | 0,09 | 0,00665833 |
| Vitamin B6 | - | 0 | 1 | 0,07398146 |
| Carotinoide | - | 0 | 3 | 0,22194438 |

In einer bevorzugten Ausführungsvariante besteht das erfindungsgemäße Kombinationspräparat aus L-Carnitin, L-Ascorbinsäure, D-alpha-Tocopherylacetat, Folsäure, Siliziumdioxid, Kalziumcarbonat, N-Acetyl-L-Cystein, Glutathion, Maisstärke, Zinkoxid, Coenzym Q10, Lycopen, pflanzliche Öle, Pteroyl-L-glutamat, Natriumselenit, Fischöl und Citrullin. Die Kapselhülle ist vorzugsweise aus Cellulose, kann jedoch auch anderer Art sein. Die pflanzlichen Öle kommen daher, dass der Rohstoff Lycopen fettlöslich ist und in ihnen gelöst angeboten wird. Statt Natriumselenit kann auch die organische Variante des L-Selenomethionins verwendet werden. Die Maisstärke ist ein Trägerstoff des sonst öligen Vitamin E.

Die positive Wirkung von Citrullin und des erfindungsgemäßen Kombinationspräparats kann sowohl in Vorbereitung für eine natürliche Schwangerschaft als auch in Vorbereitung für eine Insemination, In vitro Fertilisierung als auch Intracytoplasmische Spermieninjektion genutzt werden.

Das erfindungsgemäße Kombinationspräparat sollte zweimal täglich in Form einer Kapsel oral eingenommen werden. Die empfohlene Mindesteinnahmedauer beträgt 3 Monate.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten des Kombinationspräparats, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der beschriebenen Ausführungsvariante möglich sind, vom Schutzumfang mit umfasst.

Die den eigenständigen erfinderischen Lösungen zugrunde liegende Aufgabe kann der Beschreibung entnommen werden.

## Patentansprüche

1. Kombinationspräparat enthaltend zumindest die Wirkstoffe C-Vitamin, E-Vitamine, Zink, Selen, Acetylcystein, L-Carnitin, Glutathion, Ubiquinon-10-Komponente, Lycopen und Citrullin, wobei Citrullin in einer Tagesdosis von 200 mg bis 400 mg verabreicht wird und/oder Omega-3-Fettsäuren, zur Anwendung in einem Verfahren für die Behandlung von Oligozoospermie, Asthenozoospermie und/oder Teratozoospermie.

2. Kombinationspräparat zur Anwendung gemäß Anspruch 1, **dadurch gekennzeichnet, dass** die Omega-3-Fettsäuren Eicosapentaensäure zu Docosahexaensäure in einem Verhältnis von 3 zu 2 enthalten sind.

3. Kombinationspräparat zur Anwendung gemäß einem der Ansprüche 1 bis 2, **dadurch gekennzeichnet, dass** C-Vitamin in Mengen von 1 bis 20 Gew.-%, insbesondere 5 bis 15 Gew.-%, vorzugsweise 6 bis 10 Gew.-%, E-Vitamine in Mengen von 1 bis 20 Gew.-%, insbesondere 5 bis 15 Gew.-%, vorzugsweise 6 bis 10 Gew.-%, B9-Vitamin in Mengen von 0,005 bis 2 Gew.-%, insbesondere 0,009 bis 1 Gew.-%, vorzugsweise 0,01 bis 0,1 Gew.-%, Zink in Mengen von 0,5 bis 5 Gew.-%, insbesondere 0,8 bis 3,5 Gew.-%, vorzugsweise 1,2 bis 2,5 Gew.-%, Selen in Mengen von 0,001 bis 1 Gew.-%, insbesondere 0,004 bis 0,5 Gew.-%, vorzugsweise 0,006 bis 0,1 Gew.-%, Acetylcystein in Mengen von 0,5 bis 10 Gew.-%, insbesondere 1 bis 6 Gew.-%, vorzugsweise 2,5 bis 4,5 Gew.-%, Carnitin in Mengen von 3 bis 30 Gew.-%, insbesondere 5 bis 25 Gew.-%, vorzugsweise 10 bis 23 Gew.-%, Glutathion in Mengen von 0,5 bis 15 Gew.-%, insbesondere 1 bis 10 Gew.-%, vorzugsweise 3 bis 6 Gew.-%, Lycopen in Mengen von 0,05 bis 3 Gew.-%, insbesondere 0,08 bis 1 Gew.-%, vorzugsweise 0,2 bis 0,1 Gew.-%, und eine Ubiquinon-10-Komponente in Mengen von 0,05 bis 10 Gew.-%, insbesondere 0,1 bis 5 Gew.-%, vorzugsweise 0,8 bis 3 Gew.-%, und Omega-3-Fettsäuren in Mengen von 5 bis 50 Gew.-%, insbesondere 10 bis 40 Gew.-%, vorzugsweise 20 bis 35 Gew.-%, und/oder Citrullin in Mengen von 0,5 bis 40 Gew.-%, insbesondere 1 bis 30 Gew.-%, vorzugsweise 12 bis 20 Gew.-%, bezogen auf das Gesamtgewicht der therapeutisch wirksamen Wirkstoffe des Kombinationspräparats enthalten sind.

4. Kombinationspräparat zur Anwendung gemäß einem der Ansprüche 1 bis 3, **dadurch gekennzeichnet, dass** Kupfer, weitere Carotinoide, B6-Vitamine, B12-Vitamine enthalten sind.

5. Kombinationspräparat zur Anwendung gemäß Anspruch 4, **dadurch gekennzeichnet, dass** Kupfer in Mengen von 0,001 bis 1 Gew.-%, insbesondere 0,003 bis 0,5 Gew.-%, vorzugsweise 0,05 bis 0,1 Gew.-%, B6-Vitamine in Mengen von 0,05 bis 3 Gew.-%, insbesondere 0,08 bis 1 Gew.-%, vorzugsweise 0,2 bis 0,1 Gew.-%, B12-Vitamine in Mengen von 0,001 bis 1 Gew.-%, insbesondere 0,003 bis 0,5 Gew.-%, vorzugsweise 0,05 bis 0,1 Gew.-%, weitere Carotinoide, wie β-Carotin, α-Carotin, β-Cryptoxanthin, Lutein und Zeaxanthin in Mengen von 0,05 bis 5 Gew.-%, insbesondere 0,08 bis 1 Gew.-%, vorzugsweise 0,2 bis 0,1 Gew.-%, bezogen auf das Gesamtgewicht der therapeutisch wirksamen Wirkstoffe des Kombinationspräparats enthalten sind.

6. Kombinationspräparat zur Anwendung gemäß einem der Ansprüche 1 bis 5, **dadurch gekennzeichnet, dass** eine tägliche Dosis von zumindest 50 mg bis 200 mg Vitamin C, 10 mg bis 120 mg Vitamin E, 100 µg bis 700 µg Vitamin B9, 20 µg bis 200 µg Selen, 5 mg bis 50 mg Zink, 10 mg bis 100 mg Acetylcystein, 10 mg bis 500 mg Carnitin, 10 mg bis 100 mg Glutathion, 1 mg bis 15 mg Lycopen, 1 mg bis 50 mg Coenzym Q-10 sowie gegebenenfalls 100 mg bis 1000 mg Omega-3-Fettsäuren verabreicht wird.

7. Kombinationspräparat zur Anwendung gemäß einem der Ansprüche 1 bis 6, **dadurch gekennzeichnet, dass** eine tägliche Dosis von zumindest 100 mg Vitamin C, 100 mg Vitamin E, 500 µg Vitamin B9, 100 µg Selen, 25 mg Zink, 50 mg Acetylcystein, 300 mg Carnitin, 50 mg Glutathion, 4 mg Lycopen, 15 mg Coenzym Q 10 und 300 mg Citrullin sowie gegebenenfalls 200 mg Omega-3-Fettsäuren verabreicht wird.

8. Verwendung des Kombinationspräparates nach einem der Ansprüche 1 bis 7 als Arzneimittel, bilanzierte Diät und/oder Nahrungsergänzungsmittel.

## Claims

1. A combination compound containing at least the substances vitamin C, E vitamins, zinc, selenium, acetyl cysteine, L-carnitine, glutathione, ubiquinone-10 components, lycopene and citrulline, wherein citrulline is administered in a daily dose of 200 mg to 400 mg, and/or omega-3-fatty acids, for use in a method for the treatment of oligozoospermia, asthenozoospermia and/or teratozoospermia.

2. The combination compound for use according to claim 1, **characterized in that** it contains the omega-3-fatty acids eicosapentaenoic acid and docosahexaenoic acid in a ratio of 3 to 2.

3. The combination compound for use according to claim 1 or 2, **characterized in that** it contains vitamin C in quantities of 1 % to 20 % by weight, in particular 5 % to 15 % by weight, preferably 6 % to 10 % by weight, E vitamins in quantities of 1 % to 20 % by weight, in particular 5 % to 15 % by weight, preferably 6 % to 10 % by weight, vitamin B9 in quantities of 0.005 % to 2 % by weight, in particular 0.009 % to 1 % by weight, preferably 0.01 % to 0.1 % by weight, zinc in quantities of 0.5 % to 5 % by weight, in particular 0.8 % to 3.5 % by weight, preferably 1.2 % to 2.5 % by weight, selenium in quantities of 0.001 % to 1 % by weight, in particular 0.004 % to 0.5 % by weight, preferably 0.006 % to 0.1 % by weight, acetyl cysteine in quantities of 0.5 % to 10 % by weight, in particular 1 % to 6 % by weight, preferably 2.5 % to 4.5 % by weight, carnitine in quantities of 3 % to 30 % by weight, in particular 5 % to 25 % by weight, preferably 10 % to 23 % by weight, glutathione in quantities of 0.5 % to 15 % by weight, in particular 1 % to 10 % by weight, preferably 3 % to 6 % by weight, lycopene in quantities of 0.05 % to 3 % by weight, in particular 0.08 % to 1 % by weight, preferably 0.2 % to 0.1 % by weight, and a ubiquinone-10 component in quantities of 0.05 % to 10 % by weight, in particular 0.1 % to 5 % by weight, preferably 0.8 % to 3 % by weight, and omega 3 fatty acids in quantities of 5 % to 50 % by weight, in particular 10 % to 40 % by weight, preferably 20 % to 35 % by weight, and/or citrulline in quantities of 0.5 % to 40 % by weight, in particular 1 % to 30 % by weight, preferably 12 % to 20 % by weight, with respect to the total weight of the therapeutically effective substances of the combination compound.

4. The combination compound for use according to any one of claims 1 to 3, **characterized in that** it contains copper, other carotinoids, B6 vitamins, B12 vitamins.

5. The combination compound for use according to claim 4, **characterized in that** it contains copper in quantities of 0.001 % to 1 % by weight, in particular 0.003 % to 0.5 % by weight, preferably 0.05 % to 0.1 % by weight, B6 vitamins in quantities of 0.05 % to 3 % by weight, in particular 0.08 % to 1 % by weight, preferably 0.2 % to 0.1 % by weight, B12 vitamins in quantities of 0.001 % to 1 % by weight, in particular 0.003 % to 0.5 % by weight, preferably 0.05 % to 0.1 % by weight, other carotinoids, such as β-carotene, α-carotene, β-cryptoxanthin, lutein and zeaxanthin, in quantities of 0.05 % to 5 % by weight, in particular 0.08 % to 1 % by weight, preferably 0.2 % to 0.1 % by weight, with respect to the total weight of the therapeutically effective substances of the combination compound.

6. The combination compound for use according to any one of claims 1 to 5, **characterized in that** a daily dose of at least 50 mg to 200 mg of vitamin C, 10 mg to 120 mg of vitamin E, 100 µg to 700 µg of vitamin B9, 20 µg to 200 µg of selenium, 5 mg to 50 mg of zinc, 10 mg to 100 mg of acetyl cysteine, 10 mg to 500 mg of carnitine, 10 mg to 100 mg of glutathione, 1 mg to 15 mg of lycopene, 1 mg to 50 mg of coenzyme Q 10 as well as, optionally, 100 mg to 1000 mg of omega 3 fatty acids is administered.

7. The combination compound for use according to any one of claims 1 to 6, **characterized in that** a daily dose of at least 100 mg of vitamin C, 100 mg of vitamin E, 500 µg of vitamin B9, 100 µg of selenium, 25 mg of zinc, 50 mg of acetyl cysteine, 300 mg of carnitine, 50 mg of glutathione, 4 mg of lycopene, 15 mg of coenzyme Q 10 and 300 mg of citrulline as well as, optionally, 200 mg of omega 3 fatty acids is administered.

8. Use of the combination compound according to any one of claims 1 to 7 as a medicament, balanced diet and/or food supplement.

## Revendications

1. Préparation en association contenant au moins les principes actifs vitamine C, vitamines E, zinc, sélénium, acétylcystéine, L-carnitine, glutathion, composant ubiquinone-10, lycopène et citrulline, la citrulline étant administrée à une dose journalière de 200 mg à 400 mg, et/ou des acides gras oméga-3, pour une utilisation dans un procédé pour le traitement de l'oligozoospermie, de l'asthénozoospermie et/ou de la tératozoospermie.

2. Préparation en association pour l'utilisation selon la revendication 1, **caractérisée en ce que** les acides gras oméga-3 acide éicosapentaénoïque et acide docosahexaénoïque y sont contenus selon un rapport de 3 à 2.

3. Préparation en association pour l'utilisation selon l'une des revendications 1 à 2, **caractérisée en ce qu'**elle contient, par rapport au poids total des principes thérapeutiquement actifs de la préparation en association, la vitamine C en des quantités de 1 à 20 % en poids, en particulier de 5 à 15 % en poids, de préférence de 6 à 10 % en poids, les vitamines E en des quantités de 1 à 20 % en poids, en particulier de 5 à 15 % en poids, de préférence de 6 à 10 % en poids, la vitamine B9 en des quantités de 0,005 à 2 % en poids, en particulier de 0,009 à 1 % en poids, de préférence de 0,01 à 0,1 % en poids, le zinc en des quantités de 0,5 à 5 % en poids, en particulier de 0,8 à 3,5 % en poids, de préférence de 1,2 à 2,5 % en poids, le sélénium en des quantités de 0,001 à 1 % en poids, en particulier de 0,004 à 0,5 % en poids, de préférence de 0,006 à 0,1 % en poids, l'acétylcystéine en des quantités de 0,5 à 10 % en poids, en particulier de 1 à 6 % en poids, de préférence de 2,5 à 4,5 % en poids, la carnitine en des quantités de 3 à 30 % en poids, en particulier de 5 à 25 % en poids, de préférence de 10 à 23 % en poids, le glutathion en des quantités de 0,5 à 15 % en poids, en particulier de 1 à 10 % en poids, de préférence de 3 à 6 % en poids, le lycopène en des quantités de 0,05 à 3 % en poids, en particulier de 0,08 à 1 % en poids, de préférence de 0,2 à 0,1 % en poids, et un composant ubiquinone-10 en des quantités de 0,05 à 10 % en poids, en particulier de 0,1 à 5 % en poids, de préférence de 0,8 à 3 % en poids, et les acides gras oméga-3 en des quantités de 5 à 50 % en poids, en particulier de 10 à 40 % en poids, de préférence de 20 à 35 % en poids, et/ou la citrulline en des quantités de 0,5 à 40 % en poids, en particulier de 1 à 30 % en poids, de préférence de 12 à 20 % en poids.

4. Préparation en association pour l'utilisation selon l'une des revendications 1 à 3, **caractérisée en ce qu'**elle contient du cuivre, d'autres caroténoïdes, des vitamines B6, des vitamines B12.

5. Préparation en association pour l'utilisation selon la revendication 4, **caractérisée en ce que**, par rapport au poids total des principes thérapeutiquement actifs de la préparation en association, elle contient le cuivre en des quantités de 0,001 à 1 % en poids, en particulier de 0,003 à 0,5 % en poids, de préférence de 0,05 à 0,1 % en poids, les vitamines B6 en des quantités de 0,05 à 3 % en poids, en particulier de 0,08 à 1 % en poids, de préférence de 0,2 à 0,1 % en poids, les vitamines B12 en des quantités de 0,001 à 1 % en poids, en particulier de 0,003 à 0,5 % en poids, de préférence de 0,05 à 0,1 % en poids, les autres caroténoïdes, tels que le β-carotène, l'α-carotène, la β-cryptoxanthine, la lutéine et la zéaxanthine en des quantités de 0,05 à 5 % en poids, en particulier de 0,08 à 1 % en poids, de préférence de 0,2 à 0,1 % en poids.

6. Préparation en association pour l'utilisation selon l'une des revendications 1 à 5, **caractérisée en ce qu'**on administre une dose journalière d'au moins 50 mg à 200 mg de vitamine C, de 10 mg à 120 mg de vitamine E, de 100 µg à 700 µg de vitamine B9, de 20 µg à 200 µg de sélénium, de 5 mg à 50 mg de zinc, de 10 mg à 100 mg d'acétylcystéine, de 10 mg à 500 mg de carnitine, de 10 mg à 100 mg de glutathion, de 1 mg à 15 mg de lycopène, de 1 mg à 50 mg de coenzyme Q-10 ainsi qu'éventuellement de 100 mg à 1000 mg d'acides gras oméga-3.

7. Préparation en association pour l'utilisation selon l'une des revendications 1 à 6, **caractérisée en ce qu'**on administre une dose journalière d'au moins 100 mg de vitamine C, de 100 mg de vitamine E, de 500 µg de vitamine B9, de 100 µg de sélénium, de 25 mg de zinc, de 50 mg d'acétylcystéine, de 300 mg de carnitine, de 50 mg de glutathion, de 4 mg de lycopène, de 15 mg de coenzyme Q 10 et de 300 mg de citrulline, ainsi qu'éventuellement de 200 mg d'acides gras oméga-3.

8. Utilisation de la préparation en association selon l'une des revendications 1 à 7 en tant que médicament, régime équilibré et/ou complément alimentaire.
